# EUROPEAN PATENT SPECIFICATION

(11) **EP 0 716 647 B1**
(45) Date of publication and mention of the grant of the patent: **24.03.1999**
(21) Application number: 94925320.7
(22) Date of filing: 31.08.1994
(51) Int. Cl.: C07C 327/32, G01N 33/68

(54) **HYDRAZINO-TYPE N2S2 CHELATORS**
N2S2-CHELATOREN DES HYDRAZINO-TYPS
CHELATANTS DE TYPE HYDRAZINO A CONFIGURATION N2S2

(30) Priority: 03.09.1993 US 116504
(43) Date of publication of application: 19.06.1996
(73) Proprietor: RESOLUTION PHARMACEUTICALS INC., Mississauga, Ontario L4V 1V7 (CA)
(72) Inventor: POLLAK, Alfred, Toronto, Ontario M6B 4C6 (CA); KIRBY, Robert, A., Acton, Ontario L7J 1S4 (CA); DUFAULT, Robert, Bramalea, Ontario L6T 3V4 (CA)
(74) Representative: Sheard, Andrew Gregory
(86) International application number: PCT/CA94/00479
(87) International publication number: WO 95/06633

(56) References cited:
- EP-A- 0 417 870
- JOURNAL OF MEDICINAL CHEMISTRY, vol.29, no.10, October 1986 Washington, DC, US, pages 1933 - 1940 S. KASINA, ET AL.: 'Tissue distribution properties of technetium-99m-diamide- dimercaptide complexes and potential use as renal radiopharmaceuticals'
- JOURNAL OF NUCLEAR MEDICINE,, vol.24, no.2, February 1984 New York, US, pages 223 - 229 R.F. SCHNEIDER, ET AL.: 'N,N'-bis(S-Benz- oylmercaptoacetamido)ethylenediamine and propylenediamine ligands as renal function imaging agents. I. Alternative synthetic methods'

## Description

This invention is in the field of diagnostic imaging, and relates to chemical chelators useful in the radiolabelling of agents that target tissues of diagnostic interest.

### Background to the Invention

The art of diagnostic imaging exploits contrasting agents that in binding or localizing site selectively within the body, help to resolve the image of diagnostic interest. ⁶⁷Gallium-citrate, for example, has affinity for tumours and infected tissue and, with the aid of scanning tomography, can reveal afflicted body regions to the physician. Other contrasting agents include the metal radionuclides such as ^{99m}technetium and ^{156/188}rhenium, and these have been used to label targetting molecules, such as proteins, peptides and antibodies that localize at desired regions of the human body.

As targetting agents, proteins and other macromolecules can offer the tissue specificity required for diagnostic accuracy; yet labelling of these agents with metal radionuclides is made difficult by their physical structure. Particularly, protein and peptide targetting agents present numerous sites at which radionuclide binding can occur, resulting in a product that is labelled heterogeneously. Also, and despite their possibly large size, proteins rarely present the structural configuration most appropriate for high affinity radionuclide binding, i.e. a region incorporating four or more donor atoms that form five-membered rings. As a result, radionuclides are bound typically at the more abundant low-affinity sites, forming unstable complexes.

To deal with the problem of background binding, Paik et al (Nucl Med Biol 1985, 12:3) proposed a method whereby labelling of antibody is performed in the presence of excess DPTA (diaminetrimethylenepentaacetic acid), to mask the low affinity binding sites. While the problem of low affinity binding is alleviated by this method, actual binding of the radionuclide, in this case technetium, was consequently also very low. The direct labelling of proteins having a high proportion of cysteine residues also has been demonstrated (Dean et al.; WO 92/13,572). This approach exploits thiol groups of cysteine residues as high-affinity sites for radionuclide binding, and is necessarily limited in application to those targetting agents having the required thiol structure.

A promising alternative to the direct labelling of targetting agents is an indirect approach, in which targetting agent and radionuclide are conjugated using a chelating agent. Candidates for use as chelators are those compounds that bind tightly to the chosen metal radionuclide and also have a reactive functional group for conjugation with the targetting molecule. For use in labelling peptide and protein-based targetting agents, the chelator is ideally also peptide-based, so that the chelator/targetting agent conjugate can be synthesized in toto using peptide synthesis techniques. For utility in diagnostic imaging, the chelator desirably has characteristics appropriate for its *in vivo* use, such as blood and renal clearance and extravascular diffusibility.

In diagnostic imaging N₂S₂ chelators complexed with Tc-99m may be used as described in J. Nucl. Med., 1984, 24(2), 223-229 and J. Med. Chem., 1986, 29(10), 1933-1940. Such chelators contain the group -CH₂C(=O)CH₂- between the two nitrogen atoms.

### Summary of the Invention

The present invention provides chelators that bind diagnostically and therapeutically useful metal radionuclides, and can be conjugated to targetting agents capable of localizing at body sites of diagnostic and therapeutic interest. The chelators of the present invention are peptide analogues designed structurally to present an N₂S₂ configuration capable of binding oxo, dioxo and nitrido ions of ^{99m}technetium and ^{186/188}rhenium.

More particularly, and according to one aspect of the invention, there are provided metal radionuclide chelators of the formula: 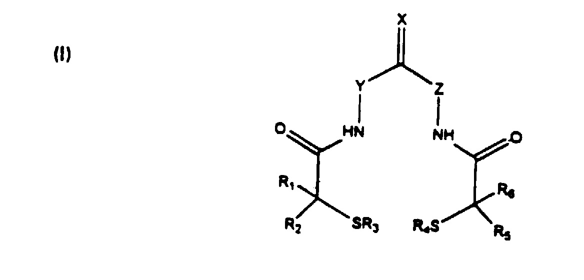 wherein
R₁, R₂, R₅ and R₆ are independently selected from H; carboxyl; C₁₋₃ alkyl; and C₁₋₃ alkyl substituted with a group selected from hydroxyl, sulfhydryl, halogen, carboxyl and aminocarbonyl;
R₃ and R₄ are independently selected from H and a sulfur protecting group;
X is selected from O, the group NH₂⁺, and the group CH₂;
Y and Z are independently selected from the group CR₁R₂ and NR₇ wherein at least one of Y and Z is NR₇; and
R₇ is selected from H, carboxyl, C₁₋₃ alkyl and C₁₋₃ alkyl substituted with a group selected from hydroxyl, carboxyl and halogen.

According to another aspect of the invention, the chelators of the above formula are provided in a form having a metal radionuclide complexed therewith.

In another aspect of the invention, the chelator is provided in a form having a conjugating group attached thereto for coupling to a diagnostically useful targetting molecule, and optionally in combination with a complexed metal radionuclide, for imaging use.

### Detailed Description of the Invention

The invention provides metal radionuclide chelators that when complexed with a radionuclide and conjugated to a targetting molecule are useful for delivering the radionuclide to a body site of therapeutic or diagnostic interest. As illustrated in the above formula, the chelators have an N₂S₂ configuration in which the radionuclide is complexed.

Terms defining the variables R₁ - R₇ as used hereinabove have the following meanings:
"lower alkyl" which refers to a straight or branched C₁-C₃ chain;
""halogen" refers to F, Cl, Br and I;
"sulfur protecting group" refers to a chemical group that inhibits oxidation, in particular those that are cleaved upon chelation of the metal. Sulfur protecting groups include known alkyl, aryl, acyl, alkanoyl, aryloyl, mercaptoacyl and organothio groups.

In preferred embodiments of the invention, the chelators conform to the above formula in which:
R₁, R_{2,} R₅ and R₆ are independently selected from H and a lower alkyl group selected from ethyl and propyl; and most preferably are all H;
R₃ and R₄ are a hydrogen atom or a sulfur protecting group selected from benzoyl, acetamidomethyl and substituted or unsubstituted tetrahydropyranyl groups;
X is selected from O, the group CH₂, and most preferably the group NH₂⁺;
Y and Z are independently selected from the groups CR₁R₂ and NR₇ wherein at least one and most preferably both of Y and Z is NR₇; and
R₇ is selected from carboxyl, lower alkyl and most preferably hydrogen.

Chelators having achiral carbon centres will advantageously alleviate the problems associated with differing biodistribution of various stereoisomers. In order to maintain achirality, a conjugating group must be attached to chelators of the invention at X. Attachment of a conjugating group at X is most stable when X is the group NH₂⁺. Accordingly, it is a preferred embodiment of the present invention to provide chelators having achiral carbon centres and having a conjugating group attached to an NH₂⁺ group at X.

In specific embodiments of the invention, the chelators conform to the above general formula wherein R₁ is H or a sulfur protecting group; R₁, R₂ R₅ and R₆ are each H; X is O, CH₂ or NH₂⁺; and Y and Z are CH₂ or NH. Specific examples include:
N,N'-bis-(S-benzoylmercaptoacetyl)-carbohydrazide and
N,N'-bis-(S-benzoylmercaptoacetyl)-1,3-diaminoguanidine hydrochloride.

For coupling to a targetting molecule, X or one of R₁, R₂, R₅ and R₆ desirably incorporates a "conjugating group", a term used hereinafter to refer to chemically reactive groups that allow for coupling of the chelator to a targetting molecule. In the preferred case where the targetting molecule is a peptide or protein, the conjugating group is reactive under conditions that do not denature or otherwise adversely affect the peptide or protein. In one embodiment of the invention, the conjugating group is reactive with a functional group of the peptide/protein such as an amino terminal group or an ε-amino group of a lysine residue so that the reaction can be conducted in a substantially aqueous solution. Useful conjugating groups include but are not limited to carboxyl groups, activated esters, a carboxy-methyl thio group, thiocyanates, amines, hydrazines, maleimides, thiols, and activated halides. In a preferred embodiment of the invention, conjugating groups are selected from methyl propanoate, carboxyl group and N-hydroxysuccinimide ester. Carboxyl conjugating groups may be activated with carbodiimide and an alcohol to form an active ester that is reactive with an amino group available on such targetting molecules as peptides and amino sugars, to form an amide linkage between the targetting molecule and the chelator conjugating group.

In certain embodiments, chelators of the present invention are provided wherein X is a CH₂ or oxo group. In a preferred embodiment, X forms an NH₂⁺ group that may have a conjugating group attached thereto forming the group NH⁺ -conjugate. A conjugating group may be introduced to this type of chelator during standard synthesis of a diaminoguanidine intermediate. Briefly, thiocarbohydrazine is converted to methyl-thiocarbohydrazine by substitution with methyl iodide. Addition of a selected amino-substituted conjugating group to the methyl-thiocarbohydrazine results in an intermediate which is subsequently employed in the preparation of chelators wherein X is NH₂⁺ having a conjugate bound thereto. A method of preparing a conjugate bound intermediate is represented below.

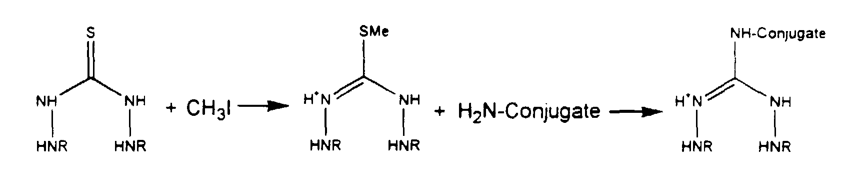

Chelators of the present invention that are symmetrical ie. in which R₁ and R₂ correspond to R₅ and R₆ respectively, can be prepared by the following general procedure. Commercially available iodoacetic acid, or a variant thereof substituted as desired, is reacted with potassium thiobenzoate yielding benzoylmercaptoacetic acid. This intermediate is then transformed into the corresponding N-hydroxysuccinimide ester using dicyclocarbodiimide in dioxane. The active ester is reacted with a selected carbohydrazide or diaminoguanidine or diaminoacetone yielding the symmetrical chelator. This method of preparing symmetrical chelators is illustrated below:

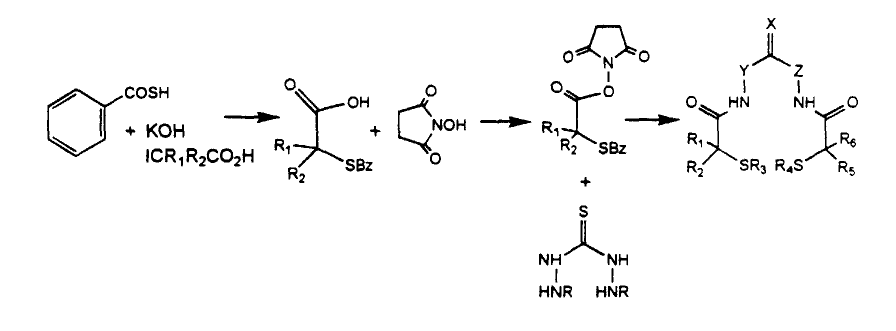

Preparation of chelators of the invention that are not symmetrical ie. in which R₁ and R₂ do not correspond to R₅ and R₆, requires steps additional to those presented in the scheme above. In a particular method, benzoylmercaptoacetyl-N-hydroxysuccinimide, prepared as described above is reacted with a selected carbohydrazide, diaminoguanidine or diaminoacetone of the general formula

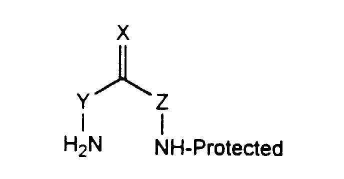

The resulting benzoylmercaptoacetyl-carbohydrazide/-diaminoguanidine/ -diaminoacetone is deprotected and subsequently reacted with a benzoylmercaptoacetyl-N-hydroxysuccinimide having the selected R₅ and R₆ groups to yield the asymmetrical N₂S₂ chelator.

In a particular embodiment of the present invention, an N-protected diaminoguanidine is prepared from a hydrazine ester in which the OR group of the ester is substituted with an N-protected hydrazine to give the mono-N-protected diaminoguanidine. N-protecting groups common to the art may be used, for example *t*-butyloxycarbonyl (*t*-Boc) or 9-fluorenylmethyloxycarbonyl (FMOC). Removal of t-Boc protecting groups can be achieved by addition of an anhydrous acid such as HCI in acetic acid while FMOC may be cleaved with piperidine and diiodomethane or piperidine dimethylformamide.

Chelators of the present invention may also be asymmetrical with respect to Y and Z. That is, one of Y or Z may be the group CR₁R₂ while the other is NR₇. This type of asymmetry may be introduced by using the intermediate 1,3-diaminoamidine or a derivative thereof in place of the intermediate 1,3-diaminoguanidine in the synthetic processes previously described.

It is to be understood that variation at R₁, R₂, R₅ and R₆ can be introduced by using variants of iodo acetic acid. For example the alpha carbon may have one or two substituents as well as iodine such as lower alkyl, substituted lower alkyl or a conjugating group. Variation at Y and Z may be introduced by using derivatives of carbohydrazide, diamino guanidine and diamino acetone. For example carbohydrazide and diamino guanidine intermediates may have carboxyl, lower alkyl, substituted lower alkyl or a conjugating group at either or both alpha nitrogens while diamino acetone may also have carboxyl; lower alkyl; substituted lower alkyl or a conjugating group at either or both alpha carbons.

A method of preparing asymmetric chelators of the invention is represented below:

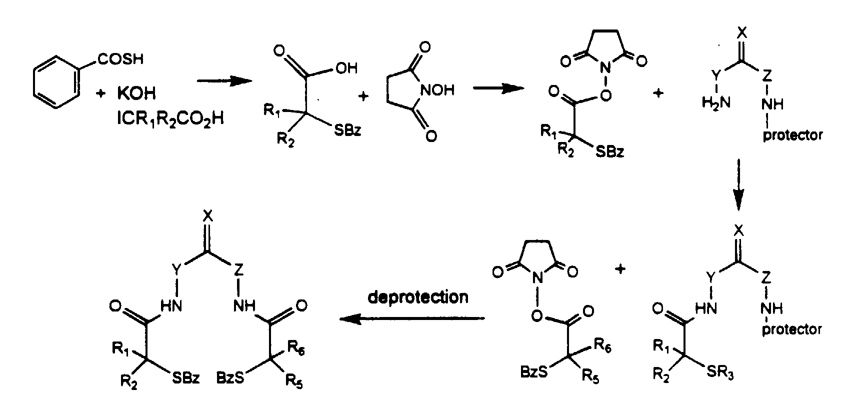

For diagnostic imaging purposes, the chelators per se may be used in combination with a metal radionuclide. Suitable radionuclides include technetium and rhenium in their various forms such as ^{99m}TcO³⁺, ^{99m}TcO₂⁺, ReO³⁺ and ReO₂⁺. Most desirably, and according to another aspect of the invention, the chelator is coupled through its conjugating group to a targetting molecule that serves to localize the chelated radionuclides at a desired site in a mammal. Examples of targetting molecules include, but are not limited to, steroids, proteins, peptides, antibodies, nucleotides and saccharides. Preferred targetting molecules include proteins and peptides, particularly those capable of binding with specificity to cell surface receptors characteristic of a particular pathology. For instance, disease states associated with over-expression of particular protein receptors can be imaged by labelling that protein or a receptor binding fragment thereof in accordance with the present invention. Targetting peptides useful to image certain medical conditions and tissues are noted below:

| for atherosclerotic plaque: | |
|---|---|
| YRALVDTLK | RALVDTLK |
| RALVDTLKFVTQAEGAK | YAKFRETLEDTRDRMY |
| AKFRETLEDTRDRMY | AALDLNAVANKIADFEL |
| YAALDLNAVANKIADFEL | YRALVDTLKFVTEQAKGA |
| RALVDTLKFVTEQAKGA | YRALVDTEFKVKQEAGAK |
| RALVDTEFKVKQEAGAK | YRALVDTLKFVTQAEGAK |

| for infections and atherosclerotic plaque: | |
|---|---|
| VGVAPGVGVAPGVGVAPG | formyl.Nleu.LF.Nleu.YK |
| VPGVGVPGVGVPGVGVPGVG | formylMIFL |
| formylMLFK | formylMLFI |
| formylMFIL | formylMFLI |
| formylMLIF | formylMILF |
| formylTKPR | VGVAPG |
| formylMLF | YIGSR |
| CH₂CO.YIGSRC | |

| for thrombus: | |
|---|---|
| NDGDFEEIPEEYLQ | NDGDFEEIPEEY(SO₃Na)LQ |
| GPRG | |

| for platelets: | |
|---|---|
| D-Phe.PRPGGGGNGDFEEIPEEYL | RRRRRRRRRGDV |
| PLYKKIIKKLLES | RGD |
| RGDS | |

for amyloid plaque (Alzheimer's disease):
EKPLQNFTLSFR

For connection to the chelator, a targetting molecule may comprise a "spacer" that serves to create a physical separation between the chelator and the targetting molecule. Suitable spacers are those that allow the targetting molecule to retain its *in vivo* localizing properties and are typically chemically inert groups such as an alkyl chain that is derivatized for coupling to the chelator. In the case where the targetting molecule is a peptide, the spacer may suitably be one or more amino acid residues. Preferably, peptidic targetting molecules incorporate spacers of from 1 to 5 amino acids such as those having chemically inert α-carbon side chains, such as glycine or β-alanine residues.

Peptide-based targetting molecules can be made using various established techniques. Because they are amenable to solid phase synthesis, alternating FMOC protection and deprotection is the preferred method of making short peptides. Recombinant DNA technology is preferred for producing proteins and long fragments thereof. The chelators of the present invention can be coupled to a targetting molecule prior to labelling with a radionuclide, a process referred to as the "bifunctional chelate" method. An alternative approach is the "prelabelled ligand" method in which the chelator is first labelled with a radionuclide and is then coupled to the targetting molecule.

Chelation of the selected radionuclide can be achieved by various methods. In a particular method, a chelator solution is first formed by dissolving the chelator optionally with a targetting molecule attached in aqueous alcohol eg. ethanol-water 1:1. The solution is degassed to remove oxygen then the thiol protecting groups are removed with a suitable reagent such as sodium hydroxide or by heating, and the resulting mixture is then neutralized with an organic acid such as acetic acid (pH 6.0-6.5). In the chelation step, sodium pertechnetate is added to the chelator solution with a reducing such as stannous chloride in an amount sufficient to reduce the technetium. The solution is mixed and left to react at room temperature and then heated on a water bath. In an alternative method, labelling can be accomplished with the chelator solution adjusted to pH 8. In this case, pertechnetate may be replaced with a solution of technetium complexed with ligands that are labile and exchangeable with the desired chelator. Suitable ligands include tartarate, citrate or heptagluconate. As a further alternative, sodium dithionite may be used as the reducing agent, and in this case the chelating solution is preferrably adjusted to pH 12-13 for the labelling step. In all cases, the labelled chelator may be separated from contaminants ^{99m}TcO₄⁻ and colloidal ^{99m}TcO₂ chromatographically, for example with a C-18 Sep Pak column activated with ethanol followed by dilute HCI. Eluting with dilute HCI separates the ^{99m}TcO₄⁻, and eluting with EtOH-saline 1:1 brings off the chelator while colloidal ^{99m}TcO₂ remains on the column.

Once prepared, the labelled chelator or chelator-targetting agent conjugate is administered to a patient by techniques well established in the art of radiodiagnostic imaging and radiotherapy. Typically a labelled chelator solution is administered by injection intravenously, intra-arterially, peritoneally or intratumorally in a pharmaceutically acceptable solution such as saline or blood plasma medium. The amount of labelled conjugate administered is dependent upon the toxicity profile of the chosen targetting molecule as well as the profile of the metal and is typically in the range of about 5-50 mCi/70 kg and more typically in the range of 25-35 mCi/70 kg. Localization of the metal *in vivo* is tracked by standard scintigraphic techniques at an appropriate time subsequent to its administration. The time at which an image may be obtained will depend in large part on the tissue distribution and clearance profile of the targetting molecule, for example most peptides will localize rapidly allowing for an image to be obtained within 3 hours and often within 1 hour.

The following examples are presented to illustrate certain embodiments of the present invention.

### Example 1 - PREPARATION OF N,N'-BIS-(S-BENZOYLMERCAPTOACETYL)-CARBOHYDRAZIDE RP-021

To a purged stirring solution at 0°C of (5.74g, 41.6mmoles) thiobenzoic acid in (100mL) ethanol was added (27.8mL, 3N, 83.2mmoles) potassium hydroxide followed by (7.70g, 41.6mmoles) iodoacetic acid in (30mL) ethanol. The solution stirred for 10 hours at room temperature under argon. The ethanol was rotavapped and the orange solid product was dissolved in (40mL) water. The solution was acidified to pH 2.0 where an orange precipitate formed. The precipitate was filtered, washed with water, and dried in vacuo to give (7.98g, 99% yield) benzoylmercaptoacetic acid.

To a stirring solution of (9.20g, 46.9mmoles) benzoylmercaptoacetic acid and (5.41g, 46.9mmoles) N-hydroxysuccinimide in (100mL) dioxane was added a solution of (9.70g, 47mmoles) dicyclohexylcarbodiimide in (40mL) dioxane. The reaction was stirred 12 hours, followed by cooling to 4°C, filtering, and rotavapping off the dioxane to a white solid. The solid was triturated with cold isopropanol, filtered, and dried in vacuo yielding 10.8g, 78% benzoylmercaptoacetyl-N-hydroxysuccinimide. 200mg was recrystallized from 500mg in hot ethyl acetate.

To a stirring solution of (1.17g, 4.0mmoles) benzoylmercaptoacetyl-N-hydroxysuccinimide in (36mL) dioxane was added a solution of (180mg, 2.0mmoles) carbohydrazide in (10mL) dioxane. After 1 hour, TLC showed a partial reaction and (404mg, 4.0mmoles) triethylamine was added. The reaction stirred 4 hours followed by rotavapping off the dioxane and adding (5mL) water to a white solid. This was filtered, washed with (5mL) water, and dried in vacuo yielding 772mg, 86.5% N,N'-bis-(S-benzoylmercatoacetyl)-carbohydrazide (m.p. 184-190 °C).

### Example 2 - PREPARATION OF N,N'-BIS-(S-BENZOYLMERCAPTOACETYL)-1,3-DIAMINOGUANIDINE HYDROCHLORIDE RP-032

To a purged stirring solution at 0°C of (5.74g, 41.6mmoles) thiobenzoic acid in (100mL) ethanol was added (27.8mL, 3N, 83.2mmoles) potassium hydroxide followed by (7.70g, 41.6mmoles) iodoacetic acid in (30mL) ethanol. The solution stirred for 10 hours at room temperature under argon. The ethanol was rotavapped and the orange solid product was dissolved in (40mL) water. The solution was acidified to pH 2.0 where an orange precipitate formed. The precipitate was filtered, washed with water, and dried in vacuo to give (7.98g, 99% yield) benzoylmercaptoacetic acid.

To a stirring solution of (9.20g, 46.9mmoles) benzoylmercaptoacetic acid and (5.41g, 46.9mmoles) N-hydroxysuccinimide in (100mL) dioxane was added a solution of (9.70g, 47mmoles) dicyclohexylcarbodiimide in (40mL) dioxane. The reaction was stirred 12 hours followed by cooling to 4°C, filtering, and rotavapping off the dioxane to a white solid. The solid was triturated with cold isopropanol, filtered, and dried in vacuo yielding 10.8g, 78% benzoylmercaptoacetyl-N-hydroxysuccinimide. 200mg was recrystallized from 500mg in hot ethyl acetate.

To a stirring solution of (200mg, 1.59mmoles) 1, 3-diaminoguanidine ·HCI in (3mL) methanol was added a solution of (936mg, 3.19mmoles) benzoylmercaptoacetyl-N-hydroxysuccinimide and (322mg, 3.19mmoles) triethylamine in (30mL) dioxane. The reaction stirred 14 hours overnight forming a fine white precipitate. The solvents were rotavapped off to a sticky white solid. The solid was triturated with cold isopropanol, filtered, washed with isopropanol, and dried in vacuo yielding 155mg, 32% N,N'-bis-(S-benzoylmercaptoacetyl)-1,3-diaminoguanidine hydrochloride (m.p. 126-128 °C).

### Example 3 - Preparation of ^{99m}Tc labelled chelators

Approximately lmg of each chelator was dissolved in 200µL saline or 200µL ethanol:water (1:1) in a tube. To the tube was added 100-300µL sodium ^{99m}Tc-pertechnetate (5-15mCi), 100µL phosphate buffer (0.25M, pH 7.4), and 200µL of a solution containing 50µL stannous chloride dihydrate and 40mg sodium tartrate dihydrate. The tube was capped tightly and placed in a boiling water bath for 10 minutes.

After cooling, the reaction mixture was loaded onto a C-18 solid-phase extraction cartridge (Sep-Pak) which had been activated with 5mL methanol and 5mL, 1mm HCl. The cartridge was washed with 5mM HCl and the eluate was collected in a test tube. The cartridge was then dried by forcing air through it. The product was eluted with 2mL ehanol:water (1:1) and collected in a separate tube. The cartridge was placed in another tube and the three tubes were assayed in a radionuclide dose calibrator (ionization chamber). The yield was calculated as the activity in the ethanol:water eluate divided by the total activity in the three tubes under the assumption that the desired product was relatively lipophilic. A less lipophilic chelator would elute partially in the acid wash and the apparent yield would be low.

| Labelling Yields | | | |
|---|---|---|---|
| | trial 1 | trial 2 | trial 3 |
| Example 1 | 57% | 59% | 39% |
| | | | |
| Example 2 | 31% | 25% | 58% |

### Example 4 - In vivo distribution

Distribution within rats of the exemplified chelators and a reference chelator was determined using established protocols. Briefly, male Wistar rats (Charles River, 200g) were anaesthetized with somnitol (40 to 50mg/kg) and 200µL of the labelled chelator (ie. 200µCi) was injected intravenously via the tail vein. Serial whole-body scintigrams were acquired after the first 10 minutes. Further images were obtained at 60 and 120 minutes, and then the rats were killed with anaesthesia and samples of organs (blood, heart, lung, liver, spleen, kidney, muscle, GI tract) were weighed and counted in either a well-type gamma counter or in a gamma dose calibrator depending on the organ. Dose calculations were made based on the assumption that rats weighed 200g and that the blood volume constituted 8% body weight. All results were corrected for the residual dose in the tail.

Each of the present chelators examined cleared relatively rapidly from the blood as desired. For the chelator of example 1, it was found that the Gl tract excretion was remarkably low in comparison with the N₂S₂ reference chelator. The Gl tract accounted for about 9% of the dose, and only about 14% remained in the blood. The chelator of example 2 localized primarily (30%) in the Gl tract and (20%) in urine, with only about 10% of the dose remaining in the blood.

The chelators of example 1 and 2 had approximately equal accumulation in the kidney (7-14%).

## Claims

1. A compound of the general formula: 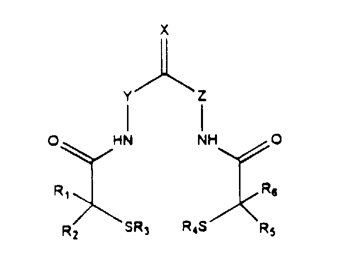 wherein
R₁, R₂, R₅ and R₆ are independently selected from H; carboxyl; C₁₋₃ alkyl; and C₁₋₃ alkyl substituted with a group selected from hydroxyl, sulfhydryl, halogen, carboxyl and aminocarbonyl;
R₃ and R₄ are independently selected from H and a sulfur protecting group;
X is selected from O, the group NH₂⁺, and the group CH₂;
Y and Z are independently selected from the group CR₁R₂ and NR₇ wherein at least one of Y and Z is NR₇; and
R₇ is selected from H, carboxyl, C₁₋₃ alkyl and C₁₋₃ alkyl substituted with a group selected from hydroxyl, carboxyl and halogen.

2. A compound according to claim 1, which is selected from:
N,N'-bis-(S-benzoylmercaptoacetyl)-carbohydrazide
N,N'-bis-(S-benzoylmercaptoacetyl)-1,3-diaminoguanidine hydrochloride.

3. A compound according to claim 1, wherein R₃ and R₄ are selected from the group consisting of a hydrogen atom, benzoyl group, an acetamidomethyl group and a substituted or unsubstituted tetrahydropyranyl group.

4. A compound according to claim 1, in a form complexed with a metal radionuclide or an oxide or nitride thereof.

5. A compound of the general formula: 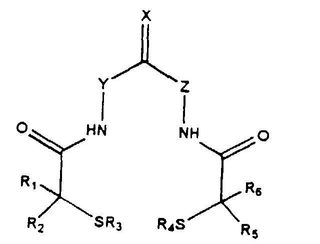 wherein
R₁, R₂, R₅ and R₆ are independently selected from H; carboxyl; C₁₋₃ alkyl; C₁₋₃ alkyl substituted with a group selected from hydroxyl, sulfhydryl, halogen, carboxyl and aminocarbonyl; and a conjugating group for coupling a targetting molecule;
R₃ and R₄ are independently selected from H and a sulfur protecting group;
X is selected from O, NH₂⁺ and CH₂ and a conjugating group for coupling a targetting molecule;
Y and Z are independently selected from the group CR₁R₂ and NR₇ wherein at least one of Y and Z is NR₇; and
R₇ is selected from H, carboxyl, C₁₋₃ alkyl and C₁₋₃ alkyl substituted with hydroxyl, carboxyl or halogen;
wherein said compound incorporates a conjugating group at one of R₁, R₂, R₅, R₆ and X.

6. A compound according to claim 5, wherein the conjugating group is selected from the group consisting of carboxyl, N-hydroxysuccinimide ester and methyl propanoate.

7. A compound according to claim 5, in a form complexed with a metal radionuclide or an oxide or nitride thereof.

8. A compound of the general formula: 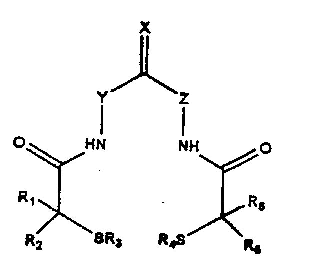 wherein
R₁, R₂, R₅ and R₆ are independently selected from H; carboxyl; C₁₋₃ alkyl; C₁₋₃ alkyl substituted with a group selected from hydroxyl, sulfhydryl, halogen, carboxyl and aminocarbonyl; and a conjugating group having a targetting molecule attached thereto;
R₃ and R₄ are independently selected from H and a sulfur protecting group;
X is selected from O, NH₂⁺ and CH₂ and a conjugating group having a targetting molecule attached thereto;
Y and Z are independently selected from the group CR₁R₂ and NR₇ wherein at least one of Y and Z is NR₇; and
R₇ is selected from H, carboxyl, C₁₋₃ alkyl and C₁₋₃ alkyl substituted with hydroxyl, carboxyl or halogen;
wherein a conjugating group having a targetting molecule attached thereto is incorporated at one of R₁, R₂, R₅, R₆ or X.

9. A compound according to claim 8, wherein the targetting molecule is a protein or peptide.

10. A compound according to claim 8, in a form complexed with a metal radionuclide or an oxide or nitride thereof.

## Patentansprüche

1. Verbindung der allgemeinen Formel: 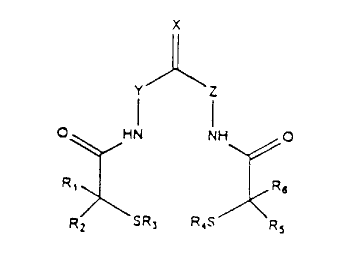 worin
R₁, R₂, R₅ und R₆ unabhängig voneinander aus H; Carboxyl; C₁₋₃-Alkyl; und C₁₋₃-Alkyl, das mit einer Gruppe, ausgewählt aus Hydroxyl, Sulfhydryl, Halogen, Carboxyl und Aminocarbonyl substituiert ist, ausgewählt sind;
R₃ und R₄ unabhängig aus H und einer Schwefelschutzgruppe ausgewählt sind;
X aus O, der Gruppe NH₂⁺ und der Gruppe CH₂ ausgewählt ist;
Y und Z unabhängig aus der Gruppe CR₁R₂ und NR₇ ausgewählt sind, wobei mindestens eine der Gruppen von Y und Z NR₇ ist; und
R₇ aus H, Carboxyl, C₁₋₃-Alkyl und C₁₋₃-Alkyl, das mit einer Gruppe, ausgewählt aus Hydroxyl, Carboxyl und Halogen substituiert ist, ausgewählt ist.

2. Verbindung nach Anspruch 1, dadurch **gekennzeichnet,** daß sie aus
N,N'-Bis-(S-benzoylmercaptoacetyl)-carbohydrazid und
N,N'-Bis-(S-benzoylmercaptoacetyl)-1,3-diaminoguanidinhydrochlorid
ausgewählt ist.

3. Verbindung nach Anspruch 1, dadurch **gekennzeichnet,** daß R₃ und R₄ aus der Gruppe, bestehend aus einem Wasserstoffatom, einer Benzoylgruppe, einer Acetamidomethylgruppe und einer substituierten oder unsubstituierten Tetrahydropyranylgruppe, ausgewählt sind.

4. Verbindung nach Anspruch 1, dadurch **gekennzeichnet,** daß sie in einer Komplexform mit einem Metallradionuclid oder einem Oxid oder Nitrid davon vorliegt.

5. Verbindung der allgemeinen Formel: 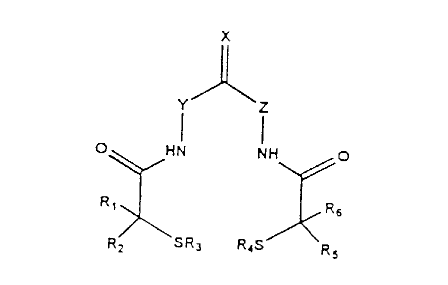 worin
R₁, R₂, R₅ und R₆ unabhängig voneinander aus H; Carboxyl; C₁₋₃-Alkyl; und C₁₋₃-Alkyl, das mit einer Gruppe, ausgewählt aus Hydroxyl, Sulfhydryl, Halogen, Carboxyl und Aminocarbonyl substituiert ist, und einer konjugierenden Gruppe für die Kopplung eines Targetmoleküls ausgewählt sind;
R₃ und R₄ unabhängig aus H und einer Schwefelschutzgruppe ausgewählt sind;
X aus O, NH₂⁺ und CH₂ und einer konjugierenden Gruppe für die Kopplung eines Targetmoleküls ausgewählt ist;
Y und Z unabhängig aus der Gruppe CR₁R₂ und NR₇ ausgewählt sind, wobei mindestens eine der Gruppen von Y und Z NR₇ ist; und
R₇ aus H, Carboxyl, C₁₋₃-Alkyl und C₁₋₃-Alkyl, das mit Hydroxyl, Carboxyl und Halogen substituiert ist, ausgewählt ist;
wobei die genannte Verbindung eine konjugierende Gruppe an einem von R₁, R₂, R₅, R₆ und X inkorporiert.

6. Verbindung nach Anspruch 5, dadurch **gekennzeichnet,** daß die konjugierende Gruppe aus der Gruppe, bestehend aus Carboxyl, N-Hydroxysuccinimidester und Methylpropanoat, ausgewählt ist.

7. Verbindung nach Anspruch 5, dadurch **gekennzeichnet,** daß sie in einer Komplexform mit einem Metallradionuclid oder einem Oxid oder Nitrid davon vorliegt.

8. Verbindung der allgemeinen Formel: 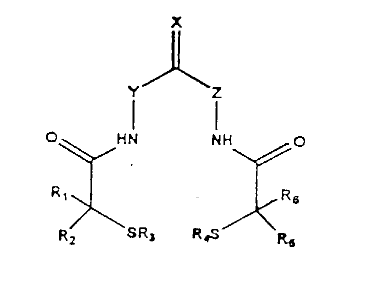 worin
R₁, R₂, R₅ und R₆ unabhängig voneinander aus H; Carboxyl; C₁₋₃-Alkyl; und C₁₋₃-Alkyl, das mit einer Gruppe, ausgewählt aus Hydroxyl, Sulfhydryl, Halogen, Carboxyl und Aminocarbonyl substituiert ist; und einer konjugierenden Gruppe mit einem angefügten Targetmolekül ausgewählt sind;
R₃ und R₄ unabhängig aus H und einer Schwefelschutzgruppe ausgewählt sind;
X aus O, NH₂⁺ und CH₂ und einer konjugierenden Gruppe mit einem angefügten Targetmolekül ausgewählt ist;
Y und Z unabhängig aus der Gruppe CR₁R₂ und NR₇ ausgewählt sind, wobei mindestens eines von Y und Z NR₇ ist; und
R₇ aus H, Carboxyl, C₁₋₃-Alkyl und C₁₋₃-Alkyl, das mit Hydroxyl, Carboxyl oder Halogen substituiert ist, ausgewählt ist;
wobei eine konjugierende Gruppe mit einem angefügten Targetmolekül an einem von R₁, R₂, R₅, R₆, oder X inkorporiert ist.

9. Verbindung nach Anspruch 8, dadurch **gekennzeichnet,** daß das Targetmolekül ein Protein oder Peptid ist.

10. Verbindung nach Anspruch 8, dadurch **gekennzeichnet,** daß sie in Komplexform mit einem Metallradionuclid oder einem Oxid oder Nitrid davon vorliegt.

## Revendications

1. Composé de formule générale : 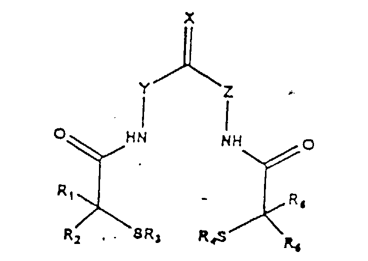 dans laquelle
R₁, R₂, R₅ et R₆ sont choisis indépendamment entre un atome d'hydrogène; un groupe carboxyle; alkyle en C₁₋₃; et alkyle en C₁₋₃ substitué par un groupe choisi parmi le groupe hydroxyle, sulfhydryle, un atome d'halogène, un groupe carboxyle et aminocarbonyle;
R₃ et R₄ sont choisis indépendamment entre un atome d'hydrogène et un groupe de protection du soufre;
X est choisi parmi un atome d'oxygène, le groupe NH₂⁺ et le groupe CH₂;
Y et Z sont choisis indépendamment entre le groupe CR₁R₂ et NR₇, au moins l'un de Y et Z étant NR₇; et
R₇ est choisi parmi un atome d'hydrogène, un groupe carboxyle, alkyle en C₁₋₃ et alkyle en C₁₋₃ substitué avec un groupe choisi entre un groupe hydroxyle, carboxyle et un atome d'halogène.

2. Composé selon la revendication 1, qui est choisi entre :
le N,N'-bis-(S-benzoylmercaptoacétyl)-carbohydrazide,
le chlorhydrate de N,N'-bis-(S-benzoylmercaptoacétyl)-1,3-diaminoguanidine.

3. Composé selon la revendication 1, dans lequel R₃ et R₄ sont choisis entre un atome d'hydrogène, un groupe benzoyle, un groupe acétamidométhyle et un groupe tétrahydropyranyle substitué ou non substitué.

4. Composé selon la revendication 1, sous une forme complexée avec un radionuclide métallique ou un oxyde ou un nitrure de celui-ci.

5. Composé de formule générale : 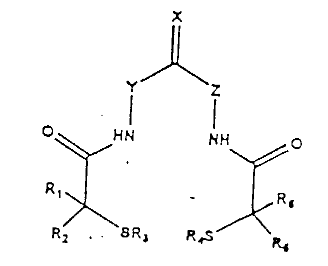 dans laquelle
R₁, R₂, R₅ et R₆ sont choisis indépendamment entre un atome d'hydrogène; un groupe carboxyle; alkyle en C₁₋₃; alkyle en C₁₋₃ substitué par un groupe choisi parmi le groupe hydroxyle, sulfhydryle, un atome d'halogène, un groupe carboxyle et aminocarbonyle; et un groupe de conjugaison pour le couplage avec une molécule cible;
R₃ et R₄ sont choisis indépendamment entre un atome d'hydrogène et un groupe de protection du soufre;
X est choisi parmi un atome d'oxygène, le groupe NH₂⁺ et le groupe CH₂ et un groupe de conjugaison pour le couplage avec une molécule cible;
Y et Z sont choisis indépendamment entre le groupe CR₁R₂ et NR₇, au moins l'un de Y et Z étant NR₇; et
R₇ est choisi parmi un atome d'hydrogène, un groupe carboxyle, alkyle en C₁₋₃ et alkyle en C₁₋₃ substitué avec un groupe hydroxyle, carboxyle ou un atome d'halogène;
dans laquelle ledit composé comprend un groupe de conjugaison sur l'un des groupes R₁, R₂, R₅, R₆ et X.

6. Composé selon la revendication 5, dans lequel le groupe de conjugaison est choisi parmi un groupe carboxyle, N-hydrosuccinimide ester et propanoate de méthyle.

7. Composé selon la revendication 5, sous une forme complexée avec un radionuclide métallique ou un oxyde ou un nitrure de celui-ci.

8. Composé de formule générale : 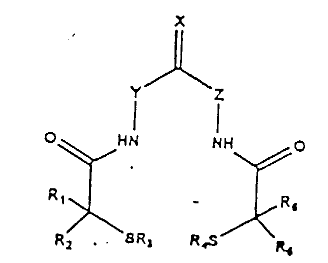 dans laquelle
R₁, R₂, R₅ et R₆ sont choisis indépendamment entre un atome d'hydrogène; un groupe carboxyle; alkyle en C₁₋₃; et alkyle en C₁₋₃ substitué par un groupe choisi parmi le groupe hydroxyle, sulfhydryle, un atome d'halogène, un groupe carboxyle et aminocarbonyle; et un groupe de conjugaison ayant une molécule cible liée à celui-ci;
R₃ et R₄ sont choisis indépendamment entre un atome d'hydrogène et un groupe de protection du soufre;
X est choisi parmi un atome d'oxygène, le groupe NH₂⁺ et le groupe CH₂ et un groupe de conjugaison ayant une molécule cible liée à celui-ci;
Y et Z sont choisis indépendamment entre le groupe CR₁R₂ et NR₇, au moins l'un de Y et Z étant NR₇; et
R₇ est choisi parmi un atome d'hydrogène, un groupe carboxyle, alkyle en C₁₋₃ et alkyle en C₁₋₃ substitué avec un groupe hydroxyle, carboxyle ou un atome d'halogène;
dans laquelle un groupe de conjugaison ayant une molécule cible liée à celui-ci est incorporé à l'un des groupes R₁, R₂, R₅, R₆ ou X.

9. Composé selon la revendication 8, dans lequel la molécule cible est une protéine ou un peptide.

10. Composé selon la revendication 8, sous une forme complexée avec un radionuclide métallique ou un oxyde ou un nitrure de celui-ci.
